## Europäisches Patentamt
## European Patent Office
## Office européen des brevets

(19)

(11) Publication number: **0 126 154**
**B1**

(12) # EUROPEAN PATENT SPECIFICATION

(45) Date of publication of patent specification: **10.08.88**

(51) Int. Cl.⁴: **A 01 N 47/36,** A 61 K 7/06, A 01 N 55/02

(21) Application number: **84900186.2**

(22) Date of filing: **21.11.83**

(86) International application number: **PCT/US83/01829**

(87) International publication number: **WO 84/02058 07.06.84 Gazette 84/14**

(54) COMPOSITION OF MATTER CONTAINING IMIDAZOLIDINYL UREA AND PYRITHIONE AND ITS DERIVATIVES.

(30) Priority: **23.11.82 US 444234**

(43) Date of publication of application: **28.11.84 Bulletin 84/48**

(45) Publication of the grant of the patent: **10.08.88 Bulletin 88/32**

(84) Designated Contracting States: **AT BE CH DE FR GB LI LU NL SE**

(56) References cited:
GB-A- 761 171
US-A-2 745 826
US-A-3 248 285

Nelson et al., "Sodium and Zinc Omadine Antimicrobials as Cosmetic Preservatives", Cosmetics and Toiletries, 96(3): 87-90 1981.

Rosen et al., "Preservation of Cosmetic Lotions with Imidazolidinyl Urea Plus Parabens", J. Soc. Cosmet. Chem. 28: 83-87 (1977).

Rosen et al., "Modern Concepts of Cosmetic Preservation", J. Soc. Cosmet. Chem. 24: 663-675 (1973).

(73) Proprietor: **SUTTON LABORATORIES, INC.**
**116 Summit Avenue**
**Chatham, NJ 07928 (US)**

(72) Inventor: **BERKE, Philip, A.**
**95 Green Avenue**
**Madison, NJ 07940 (US)**
Inventor: **ROSEN, William, E.**
**86 Canoe Brook Parkway**
**Summit, NJ 07901 (US)**

(74) Representative: **Skailes, Humphrey John et al**
**Frank B. Dehn & Co. Imperial House 15-19 Kingsway**
**London WC2B 6UZ (GB)**

EP 0 126 154 B1

**Description**

Background of the invention

The present invention is directed to a compositiom of matter which exhibits synergistic activity against contaminating microorganisms.

Pyrithione, also identified as 2-mercaptopyridine-1-oxide and 1-hydropyridine-2-thione and given CAS registry numbers 1121-30-8 and 1121-31-9, and its derivatives are known to be active against a variety of microorganisms. They, therefore, have been used in a variety of products to inhibit contamination by microorganisms including cosmetics, lubricants, and water-based paints. The most commonly used derivatives of pyrithione are the alkali metal and heavy metal salts, principally the sodium and zinc salts. See, Nelson and Hyde, "Sodium and Zinc Omadine® Antimicrobials as Cosmetic Preservatives", *Cosmetics & Toiletries,* Vol. 96, No. 3, p 87. However, other derivatives such as pyrithione disulfide and the magnesium sulfate adduct thereof have also been proposed as antimicrobial agents. Further details concerning the manufacture and use of pyrithione and its derivatives are found in United States Patent No. 2,745,826 and British Patent Specification No. 761,171.

Imidazolidinyl urea is the name given to a bacteriocidal compound prepared as described in Example IV of United States Patent No. 3,248,285 by refluxing a mixture of 600 g of allantoin (3,8 moles), 450 g (5,5 moles) of 37% aqueous formaldehyde solution and 12 g of NaOH for one hour and has been given the CAS registry number 39236-46-9. This compound has been widely used to preserve cosmetics, agricultural products and foodstuffs, paints, lubricants, plastics and textiles from bacteria.

The present invention is predicated upon the discovery that a composition of matter based on the combination of imidazolidinyl urea and pyrithione and/or its derivatives exhibits synergistic activity against the microorganism C. albicans, a commonly occurring and problematic microbial. The composition, thus, provides a new and effective means for protecting perishable products from the harmful effects of contaminating microorganisms.

Summary of the invention

The composition of the invention comprises more than 50% by weight based upon the total weight of the imidazolidinyl urea and pyrithione components of the composition of imidazolidinyl urea and less than 50% by weight based upon the total weight of the imidazolidinyl urea and pyrithione components of pyrithione and/or its derivatives.

The pyrithione component may be selected from pyrithione, alkali metal salts of pyrithione, heavy metal salts of pyrithione, pyrithione disulfide and mixtures thereof. Particularly preferred compositions are those containing sodium or zinc pyrithione and imidazolidinyl urea.

The composition of the invention may be used to preserve a variety of liquid and solid products from microbial contamination including, but not limited to, lotions, creams, soaps, shampoos and hair conditioners, lubricants, plastics, agricultural products and foodstuffs, pharmaceutical products and medicaments.

Detailed description of the invention

Since imidazolidinyl urea and pyrithione and its derivatives can be manufactured as powdered solids, the composition of the invention may be prepared as a powder by simply mixing the individual components together in the appropriate proportions. Alternatively, aqueous solutions can be prepared from imidazolidinyl urea, which is highly water soluble and the pyrithione derivatives which are also water soluble, such as sodium pyrithione. Aqueous dispersions may be prepared from non-water soluble pyrithiones, such as zinc pyrithione. Other solvents can be used to prepare liquid formulations of the composition as, for example, propylene glycol. Of course, the choice of solvent will depend upon the solubility characteristics of the particular pyrithione(s) used in the composition.

As shown in the following Table, the combination of a pyrithione and imidazolidinyl urea exhibits synergistic antimicrobial activity, in a challenge test using Candida albicans (ATCC 10231):

| Test solution | Concentration | Results on subculture after 72 hour incubation period |
|---|---|---|
| Imidazolidinyl urea | 0.3% | + |
| | 0.6% | + |
| Sodium pyrithione | 0.005% | + |
| | 0.006% | + |
| | 0.007% | + |
| | 0.008% | + |
| | 0.010% | + |
| Imidazolidinyl urea plus Sodium pyrithione | (0.3%) (0.005%) | 0 |

+ representing growth of microorganisms on subculture (i.e., microbial survival in test solution)
0 representing no growth on subculture (i.e. complete kill in test solution) The challenge test was conducted as follows:

1. 4.5 ml of the undiluted compound was placed into a 16×150 mm test tube.

2. 0.5 ml of a 24 hour Trypticase Soy Broth (TSB) culture was added to the compound.

3. The challenged compound was allowed to sit at room temperature for 72 hours, after which a count was made on the sample to recover the viable cells, using standard pour plate procedure and trypticase soy agar pour plates. The remainder of the sample was added to 100 ml of TSB for a sterility test.

4. All subculture media were incubated at 35°C for 48 hours and then observed for growth.

5. All negative media were challenged with a low number of organisms to assure that the media was growth-promoting.

The challenge test demonstrates that although 0.6% imidazolidinyl urea or 0.01% sodium pyrithione failed to kill C. albicans, a mixture of half of each, i.e., 0.3% imidazolidinyl urea and 0.005% sodium pyrithione achieved a complete kill.

The actual microbial counts make clear that the combination of imidazolidinyl urea and pyrithione is indeed synergistic. All challenges involved starting (challenge) concentrations of approximately $10^6$ microorganisms per ml of test solution. After 72 hours in either 0.3% imidazolidinyl urea or 0.005% sodium pyrithione, the concentration of microorganism was unchanged within experimental error, certainly less decrease than a factor of 10. The combination test solution would therefore be expected to have, after 72 hours, concentrations of microorganisms either unchanged or at most decreased by a factor of 20. Yet the mixture of imidazolidinyl urea and sodium pyrithione, in fact, reduced the concentration of microorganism from $10^6$ to 0 a factor of 1,000,000.

The synergistic activity of the composition of the invention makes it a highly attractive preservative for a wide variety of products which are susceptible to contamination by microorganisms.

The composition of the invention is particularly attractive for use in cosmetic products. It is readily incorporated in aqueous-based products, emulsions and solid formulations. Moreover, because of its synergistic activity, the composition is effective in applications where the individual components are by themselves ineffective. Typical cosmetic products effectively preserved by the composition of the invention are creams, lotions, shampoos, hair conditioners, eyeliners, and other eye make-up products.

In addition to cosmetics, the composition of the invention may be used to preserve almost any substance which is susceptible to microbial contamination including foodstuffs and pharmaceutical formulations. While effective amounts will vary depending upon the product being preserved, generally the amount of the composition incorporated into a product will not exceed 1.0% by weight. In this amount, the pyrithione component(s) will generally be present in an amount from about 0.025 to 0.1%, the remainder of the composition being imidazolidinyl urea.

The invention, therefore, also relates to a method of preserving products susceptible to microbial contamination by incorporating therein an effective amount of the composition of the invention, and the products so preserved.

While the invention has now been described in terms of certain preferred embodiments, one skilled in the art will readily appreciate that various modifications, changes, omissions and substitutions may be made without departing from the spirit thereof. It is intended, therefore, that the present invention be limited solely by the scope of the following claims.

**Claims**

1. A composition of matter useful as a preservative in products susceptible to microbial contamination comprising more than 50% by weight of imidazolidinyl urea obtainable by refluxing a mixture of 600 g of allantoin (3,8 moles) 450 g (5,5 moles) of 37% aqueous formaldehyde solution and 12 g of NaOH for one hour and less than 50% by weight of a 2-mercapto-pyridine-1-oxide (pyrithione) component selected from pyrithione, alkali metal salts of pyrithione, heavy metal salts of pyrithione, pyrithione disulfide and mixtures thereof, said percentages being based on the total weight of the imidazolinyl urea and the pyrithione component(s).

2. The composition as claimed in Claim 1, wherein said derivatives of pyrithione are sodium pyrithione and zinc pyrithione.

3. A product containing up to 1.0% by weight of the composition of Claim 1 or Claim 2.

4. The product of Claim 3, wherein said product is a cosmetic product.

5. The product of Claim 3, wherein said product is an agricultural product.

6. The product of Claim 3, wherein said product is a foodstuff.

7. The product of Claim 3, wherein said product is a pharmaceutical product.

8. The product of Claim 3, wherein said product is a medicament.

9. A method of preserving a product susceptible to microbial contamination comprising incorporating in said product up to 1.0% by weight of the composition of Claim 1.

10. The method of Claim 9, wherein said composition comprises sodium pyrithione as the pyrithione component.

11. The method of Claim 9, wherein said composition comprises zinc pyrithione as the pyrithione component.

12. The method of Claim 9, wherein said product is a cosmetic product.

13. The method of Claim 9 wherein said product is an agricultural product.

14. The method of Claim 9, wherein said product is a foodstuff.

15. The method of Claim 9, wherein said product is a pharmaceutical product.

16. The method of Claim 9, wherein said product is a medicament.

**Patentansprüche**

1. Zusammensetzung, die als Konservierungsmittel in Produkten nützlich ist, die für mikrobielle Verunreinigung empfindlich sind, umfassend mehr als 50 Gew.-% Imidazolinylharnstoff, der erhältlich ist durch Rückflußerhitzen eines Gemisches von 600 g Allantoin (3,8 Mol), 450 g (5,5 Mol) 37%-iger wäßriger Formaldehydlösung und 12 g NaOH während einer Stunde und weniger als 50 Gew.-% einer 2-Mercapto-pyridin-1-oxid-(Pyrithion)-komponente, ausgewählt aus Pyrithion, Alkalimetallsalzen von Pyrithion, Schwermetallsalzen von Pyrithion, Pyrithiondisulfid und Mischungen davon, wobei diese Prozentsätze auf das Gesamtgewicht des Imidazolinylharnstoffs und der Pyrithionkomponente(n) bezogen ist.

2. Zusammensetzung gemäß Anspruch 1, worin diese Derivate des Pyrithions Natriumpyrithion und Zinkpyrithion sind.

3. Ein Produkt, enthaltend bis zu 1 Gew.-% der Zusammensetzung gemäß Anspruch 1 oder Anspruch 2.

4. Produkt des Anspruchs 3, worin diese Produkt ein kosmetisches Produkt ist.

5. Produkt gemäß Anspruch 3, worin diese Produkt ein Landwirtschaftliches Produkt ist.

6. Produkt gemäß Anspruch 3, worin diese Produkt ein Nahrungsmittel ist.

7. Produkt gemäß Anspruch 3, worin diese Produkt ein pharmazeutisches Produkt ist.

8. Produkt gemäß Anspruch 3, worin diese Produkt ein Arzneimittel ist.

9. Verfahren zum Konservieren eines Produkts, das gegenüber mikrobieller Verunreinigung bzw. Verseuchung empfindlich ist, umfassend die Einverleibung bis zu 1,0 Gew.-% der Zusammensetzung gemäß Anspruch 1 in dieses Produkt.

10. Verfahren gemäß Anspruch 9, wobei diese Zusammensetzung Natriumpyrithion als Pyrithionkomponente umfaßt.

11. Verfahren gemäß Anspruch 9, wobei diese Zusammensetzung Zinkpyrithion als Pyrithionkomponente umfaßt.

12. Verfahren gemäß Anspruch 9, wobei dieses Produkt ein kosmetisches Produkt ist.

13. Verfahren gemäß Anspruch 9, wobei dieses Produkt ein landwirtschaftliches Produkt ist.

14. Verfahren gemäß Anspruch 9, wobei dieses Produkt ein Nahrungsmittel ist.

15. Verfahren gemäß Anspruch 9, wobei dieses Produkt ein pharmazeutisches Produkt ist.

16. Verfahren gemäß Anspruch 9, wobei dieses Produkt ein Arzneimittel ist.

**Revendications**

1. Composition de matière convenant à titre de conservateur pour des produits susceptibles de subir une contamination microbienne, comprenant plus de 50% en poids d'imidazolidinylurée que l'on peut obtenir par le chauffage au reflux, pendant une heure, d'un mélange de 600 g d'allontoïne (3,8 moles), de 450 g (5,5 moles) d'une solution aqueuse à 37% de formaldéhyde et de 12 g de NaOH et moins de 50% en poids d'un composant du type 1-oxyde de 2-mercaptopyridine (pyrithione), choisi parmi la pyrithione, les sels de métaux alcalins de la pyrithione, les sels de métaux lourds de la pyrithione, le bisulfure de pyrithione et leurs mélanges, les pourcentages précités étant basés sur le poids total de l'imidazolinylurée et du ou des composant(s) du type pyrithione.

2. Composition suivant la revendication 1, caractérisée en ce que les dérivés précités de la pyrithione sont le sel de sodium de la pyrithione et le sel de zinc de la pyrithione.

3. Produit contenant jusqu'à 1,0% en poids de la composition suivant la revendication 1 ou la revendication 2.

4. Produit suivant la revendication 3, caractérisé en ce qu'il est une composition cosmétique.

5 Produit suivant la revendication 3, caractérisé en ce qu'il est une composition agricole.

6. Produit suivant la revendication 3, caractérisé en ce qu'il est une composition alimentaire.

7. Produit suivant la revendication 3, caractérisé en ce qu'il est une composition pharmaceutique.

8. Produit suivant la revendication 3, caractérisé en ce qu'il est un médicament.

9. Procédé pour conserve un produit susceptible de subir une contamination microbienne, caractérisé en ce que l'on incorpore au produit en question jusqu'à 1,0% en poids de la composition suivant la revendication 1.

10. Procédé suivant la revendication 9, caractérisé en ce que la composition en question comprend le sel de sodium de la pyrithione à titre de composant du type pyrithione.

11. Procédé suivant la revendication 9, caractérisé en ce que la composition en question comprend le sel de zinc de la pyrithione à titre de composant du type pyrithione.

12. Procédé suivant la revendication 9, caractérisé en ce que le produit en question est une composition cosmétique.

13. Procédé suivant la revendication 9, caractérisé en ce que le produit en question est une composition agricole.

14. Procédé suivant la revendication 9, caractérisé en ce que le produit en question est une composition alimentaire.

15. Procédé suivant la revendication 9, caractérisé en ce que le produit en question est une composition pharmaceutique.

16. Procédé suivant la revendication 9, caractérisé en ce que le produit en question est un médicament.